# EUROPEAN PATENT SPECIFICATION

(11) **EP 4 233 504 B1**
(45) Date of publication and mention of the grant of the patent: **05.11.2025**
(21) Application number: 23157013.6
(22) Date of filing: 16.02.2023
(51) Int. Cl.: A01C 5/06, A01B 79/00, A01C 7/00, A01C 7/20, G01N 33/24

(54) **COMBINATION OF A SOIL DENSITY DETECTION SYSTEM AND AGRICULTURAL IMPLEMENT**
KOMBINATION EINES BODENDICHTEERKENNUNGSSYSTEMS UND LANDWIRTSCHAFTLICHES ARBEITSGERÄT
COMBINAISON D'UN SYSTÈME DE DÉTECTION DE DENSITÉ DE SOL ET OUTIL AGRICOLE

(30) Priority: 23.02.2022 US 202217678409
(43) Date of publication of application: 30.08.2023
(73) Proprietor: Deere & Company, Moline, IL 61265 (US)
(72) Inventor: Rains, Gerald E., Mannheim (DE); Peterson, James R., Mannheim (DE)
(74) Representative: Reichert, Christian

(56) References cited:
- US-A1- 2015 264 857
- US-A1- 2020 128 723
- US-A1- 2020 281 111

## Description

### BACKGROUND

Ground working implements are often used in fields to work the soil, such as for ground conditioning and planting.

For example, US 2020/0128723 A1 discloses an agricultural planting machine with devices, systems and method for active control of ground engaging elements based on soil conditions. Various implementations utilize one or more sensor inputs and/or database or map inputs to adjust gauge or closing wheel down force applied by a row unit to account for the moisture of the soil being planted in real time.

Further, in US 2015/0264857 A1, an agricultural planter includes systems, methods, and apparatus for providing down force pressure at row units of the planter. The row units may include an electric linear actuator connected to linkages of the row units to provide and maintain a down force pressure for the row unit. The linkage may also be removed and replaced with a strut or like mechanism to apply a direct down force pressure to components of the row unit. One or more sensors can be included to obtain information related to the ground to automatically adjust the amount of down force provided based upon a ground characteristic in order to maintain a substantially uniform furrow depth.

In US 2020/0281111 A1, a row unit for a seeding machine includes a ground view sensor coupled to the frame. The ground view sensor is operable to sense the furrow and generate depth signals corresponding to actual sensed depth of the furrow. The row unit also includes a controller configured to receive the signals, and a downforce adjustment mechanism coupled to the frame and to the controller. The controller is configured to activate the downforce adjustment mechanism to adjust a downforce on the frame based on the signals received by the controller.

The tools attached to the implements engage the ground, such as to turn, open, and/or close the soil. Soil conditions can alter the ability for ground working tools to dig into the ground, particularly when the soil is compacted, dense, or otherwise difficult to engage. Therefore, along with the design of the tools, a supplemental downward force can be applied to the implements to assist to tools in reaching a desired depth in the ground. For example, seeders are used to plant crops, where a furrow opener is used to open a furrow, a seed is planted, and the furrow is subsequently closed. In this example, the seeds are planted at a target depth that improves germination and growth. Therefore, a downward force can be applied to the implement to achieve the target depth based on soil conditions.

### SUMMARY

This Summary is provided to introduce a selection of concepts in a simplified form that are further described below in the Detailed Description. This Summary is not intended to identify key factors or essential features of the claimed subject matter, nor is it intended to be used to limit the scope of the claimed subject matter. The invention is defined by the appended claims.

According to the invention, there is a soil density detection system. The soil density detection system comprises at least one soil-density sensing device disposed on an agricultural implement traveling in a forward direction of travel, the at least one soil-density sensing device comprising a non-contact sensor that detects soil density in at least a portion of a target area (e.g., without contacting the soil) in front of the agricultural implement, and generates data, such as in the form of a signal or image indicative of the detected soil density. Such an agricultural implement might be understood as a ground working implement with row units, in particular a seeder or seeder implement.

The soil density detection system further comprises a control device operatively coupled with the at least one soil-density sensing device, the control device comprising: a data processor (e.g., a CPU processor); data memory storage (e.g., RAM, ROM memory), operatively coupled with the processor; and soil density detection logic stored in the memory storage of the control device, where the soil density detection logic is executable by the processor to determine the soil density in at least a portion of the target area. The control device is configured to generate the data indicative of the soil density, based at least upon the detected soil density throughout the target area.

In one implementation, the control device is also configured to provide an actuator adjustment command to a coupled actuator system based at least upon the data indicative of the soil density detected in the target area. In this implementation, the actuator adjustment command is indicative of an amount of actuator force (e.g., additional, less, same) is applied to the agricultural implement.

According to the invention, the soil-density sensing device is configured to detect soil density in at least a portion of the target area defined by a range of detection angles measured relative to a line normal to the ground. The detection angle ranges from about 15° to about 25° relative to the normal line. The soil density detection system further comprises a control device that is operatively coupled with the at least one soil-density sensing device, wherein the control device further operably coupled with a global positioning system (GPS) for identifying data indicative of a location of the sensed soil density. The control device can be configured to generate a soil density profile based at least upon the data indicative of the soil density detected in the target area, and identified by the GPS data.

To the accomplishment of the foregoing and related ends, the following description and annexed drawings set forth certain illustrative aspects and implementations. These are indicative of but a few of the various ways in which one or more aspects may be employed. Other aspects, advantages and novel features of the disclosure will become apparent from the following detailed description when considered in conjunction with the annexed drawings.

### BRIEF DESCRIPTION OF THE DRAWINGS

What is disclosed herein may take physical form in certain parts and arrangement of parts, and will be described in detail in this specification and illustrated in the accompanying drawings which form a part hereof and wherein:
FIGURE 1 is component diagram illustrating a side view of an example work vehicle with a ground working implement upon which a proactive soil density detection system can be used according to example implementations of the present disclosure.
FIGURE 2 is a component diagram illustrating one implementation of at least a portion of a ground working implement for use with the soil density detection system disclosed herein.
FIGURE 3 is a component diagram illustrating one implementation of at least a portion of a ground working implement for use with the soil density detection system disclosed herein.
FIGURE 4 is a schematic diagram illustrating one implementation for arranging a soil-density sensing device of the soil density detection system disclosed herein.
FIGURE 5 is a schematic diagram illustrating and example control device configured to determine variations in soil density throughout an area of interest in accordance with this disclosure.
FIGURE 6 is a flow diagram illustrating an example implementation of a general method for adjusting downward force of the row unit based upon detected soil density

### DETAILED DESCRIPTION

The claimed subject matter is now described with reference to the drawings, wherein like reference numerals are generally used to refer to like elements throughout. In the following description, for purposes of explanation, numerous specific details are set forth in order to provide a thorough understanding of the claimed subject matter. It may be evident, however, that the claimed subject matter may be practiced without these specific details. In other instances, structures and devices are shown in block diagram form in order to facilitate describing the claimed subject matter.

The word "exemplary" is used herein to mean serving as an example, instance or illustration. Any aspect or design described herein as "exemplary" is not necessarily to be construed as advantageous over other aspects or designs. Rather, use of the word exemplary is intended to present concepts in a concrete fashion. As used in this application, the term "or" is intended to mean an inclusive "or" rather than an exclusive "or." That is, unless specified otherwise, or clear from context, "X employs A or B" is intended to mean any of the natural inclusive permutations. That is, if X employs A; X employs B; or X employs both A and B, then "X employs A or B" is satisfied under any of the foregoing instances. Further, at least one of A and B and/or the like generally means A or B or both A and B. In addition, the articles "a" and "an" as used in this application and the appended claims may generally be construed to mean "one or more" unless specified otherwise or clear from context to be directed to a singular form.

Although the subject matter has been described in language specific to structural features and/or methodological acts, it is to be understood that the subject matter defined in the appended claims is not necessarily limited to the specific features or acts described above. Rather, the specific features and acts described above are disclosed as example forms of implementing the claims. Of course, those skilled in the art will recognize many modifications may be made to this configuration without departing from the scope or spirit of the claimed subject matter.

Also, although the disclosure has been shown and described with respect to one or more implementations, equivalent alterations and modifications will occur to others skilled in the art based upon a reading and understanding of this specification and the annexed drawings. The disclosure includes all such modifications and alterations and is limited only by the scope of the following claims. In particular regard to the various functions performed by the above described components (e.g., elements, resources, etc.), the terms used to describe such components are intended to correspond, unless otherwise indicated, to any component which performs the specified function of the described component (e.g., that is functionally equivalent), even though not structurally equivalent to the disclosed structure which performs the function in the herein illustrated exemplary implementations of the disclosure.

In addition, while a particular feature of the disclosure may have been disclosed with respect to only one of several implementations, such feature may be combined with one or more other features of the other implementations as may be desired and advantageous for any given or particular application. Furthermore, to the extent that the terms "includes," "having," "has," "with," or variants thereof are used in either the detailed description or the claims, such terms are intended to be inclusive in a manner similar to the term "comprising."

Furthermore, in detailing the disclosure, terms of direction, such as "forward," "rear," "front," "back," "lateral," "horizontal," and "vertical" may be used. Such terms are defined, at least in part, with respect to the direction in which the work vehicle or implement travels during use. The term "forward" and the abbreviated term "fore" (and any derivatives and variations) refer to a direction corresponding to the direction of travel of the work vehicle, while the term "aft" (and derivatives and variations) refer to an opposing direction. The term "fore-aft axis" may also reference an axis extending in fore and aft directions. By comparison, the term "lateral axis" may refer to an axis that is perpendicular to the fore-aft axis and extends in a horizontal plane; that is, a plane containing both the fore-aft and lateral axes. The term "vertical," as appearing herein, refers to an axis or a direction orthogonal to the horizontal plane containing the fore-aft and lateral axes.

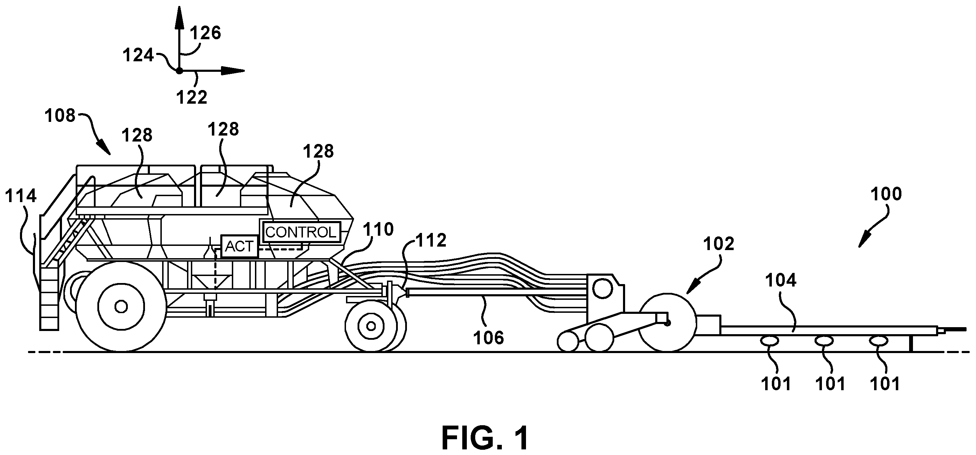

Referring now to the drawings, FIGURE 1 illustrates a soil density detection system 100 comprising one or more soil-density sensing devices 101 attached in front of one or more agricultural row units 102 (e.g., a row seeder unit). The one or more soil-density sensing devices 101 can be configured to detect the density of soil in at least a portion of an area of interest (e.g., a target area) within which the row unit 102 is targeted to operate, and to generate data indicative of a signal or image (e.g., soil density profile or map) representative of the detected soil density. The one or more soil-density sensing devices 101 can comprise non-contact sensors. For example, the sensor 101 can be disposed on the agricultural implement being driven forward (e.g., at a front of a seeder), at a location that detects soil density prior to a soil opening portion of the implement crosses that location. In this way, the soil density can be determined prior to use of the device that opens the soil (e.g., to plant a seed).

In this implementation, the one or more soil-density sensing devices 101 are mounted on a tool bar 104 that can be hitched to a tractor, either directly or indirectly, to pull the row unit 102 across a field in a direction of travel substantially parallel to a longitudinal axis 122. In other implementations, the one or more soil-density sensing devices 101 can be mounted on the tractor pulling the row unit 102 and, in particular implementations, may be disposed on a front portion of the row unit 102 provided that the soil-density sensing devices 101 are arranged so as to detect variations in soil density before the row unit 102 crosses the area (e.g., in front of the row unit) as will be described in more detail below.

In this implementation, a work vehicle 108 is coupled to the row unit 102 by a tow bar 106. The tow bar 106 extends from, and is connected to, a frame 110 of the work vehicle 108 at a front end 112 of the work vehicle 108. The work vehicle 108 may be configured to deliver a commodity (e.g., seed, fertilizer, and/or another particulate or granular commodity) stored in one or more containers 128 on the work vehicle 108 to the row unit 102. In some implementations, the work vehicle 108 and row unit 102 coupling may be towed by another vehicle, such as a tractor (not shown). In this implementation, the row unit 102 may be connected to the towing vehicle (e.g., tractor) via tool or tow bar 104 such that the row unit 102 is disposed between the work vehicle 108 and the towing vehicle with respect to the longitudinal axis 122. In other implementations, the work vehicle 108 may be a self-propelled vehicle. In these implementations, the row units 102 may be disposed behind the work vehicle 108 and/or the row units 102 may be directly connected to the work vehicle 108 (i.e., directly connected to the frame 110 of the work vehicle 108) without departing from the scope of the present disclosure.

In some implementations, multiple row units 102 may be connected together and arranged in series along a lateral axis 124 (e.g., extends into and out of the page). Thus, although only one row unit 102 is shown in FIGURE 1, it will be appreciated that similar row units 102 may be included and disposed in series along the lateral axis 124 extending between opposite lateral sides of work vehicle 108.

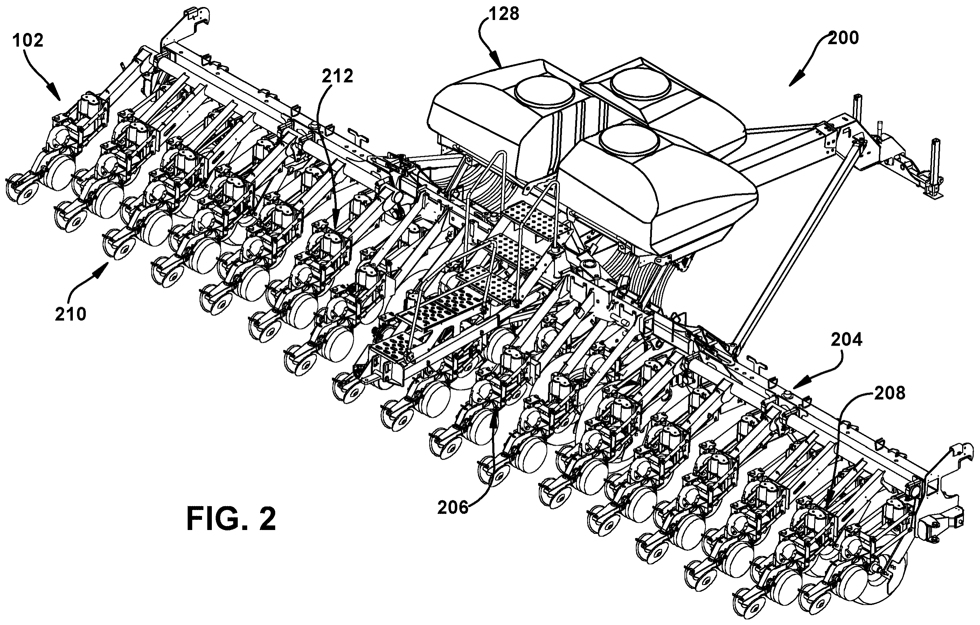

Referring now to FIGURES 2-3, there are component diagrams illustrating an example implementation of one or more portions of a ground working implement. FIGURE 2 is a component diagram illustrating an example implementation of a ground working implement 200 (e.g., a seeder implement) comprising a single row seeder unit, or simply row unit 102, such as that shown attached to the work vehicle 108 in FIGURE 1. As an example, the seeder implement 200 can be towed behind a vehicle, such as a tractor, during a seeding operation in a field.

The row unit 102 can be operably (e.g., during operation) engaged with the stationary frame 204 of the seeder implement 200. The row unit 102 can comprise a moveable frame 206, which is configured to move (e.g., up and down) during operation, that can be operably coupled to the stationary frame 204 by a linkage 208. In some implementations, the linkage 208 can comprise a four-bar linkage 208 that can be used to keep the movable frame 206 substantially parallel to the stationary frame 204 as it moves up and down.

The row unit 102 may comprise one or more actuators 212 configured to adjust the downward force of the row unit 102 against the soil. To increase the downward force in excess of the row unit weight, or to be able to adjust the force, hydraulic and/or pneumatic actuators (and/or one or more springs) can be added to the row unit 102 to urge the row unit 102 downwardly with a controllable force. The one or more actuators 212 may also be used to lift the row unit 102 off the ground for transport or to maintain seed depth by adjusting downforce to account for variations in soil density.

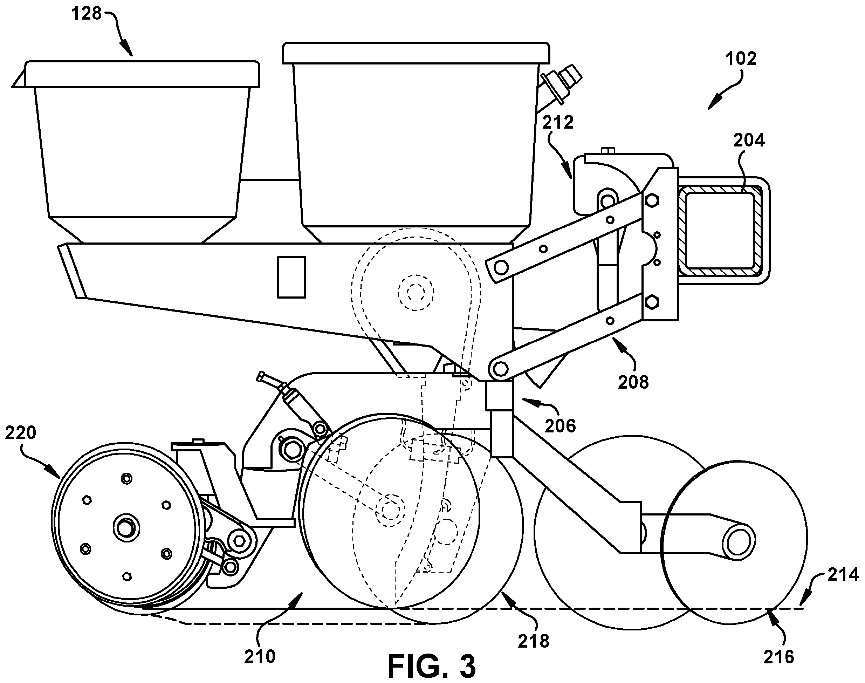

Referring now to FIGURE 3, each row unit 102 may include features for respectively tilling the soil (e.g., clearing wheels 216), opening a furrow in the soil (e.g., opener 218), depositing the commodity into the furrow, and closing the furrow (e.g., closing wheels 220) as the row unit 102 is advanced across a field (e.g., pulled) by a tractor or work vehicle 108. That is, for example, a ground working implement, such as the row unit 102, can be configured to utilize ground working tools 210 (collectively, 216, 218, and 220) to manipulate the ground (e.g., dig into the soil), such as to create a trench to place a seed, and cover the trench with earth.

Variations in soil density may impact the ground working tool's ability to trench to the desired depth. In this example, a downward force application device, for example an actuator 212, may apply downward force to the tools to drive them to the desired depth with the amount of force exerted at least based upon differences in soil density detected by the soil-density sensing devices 101 as will be explained in detail below.

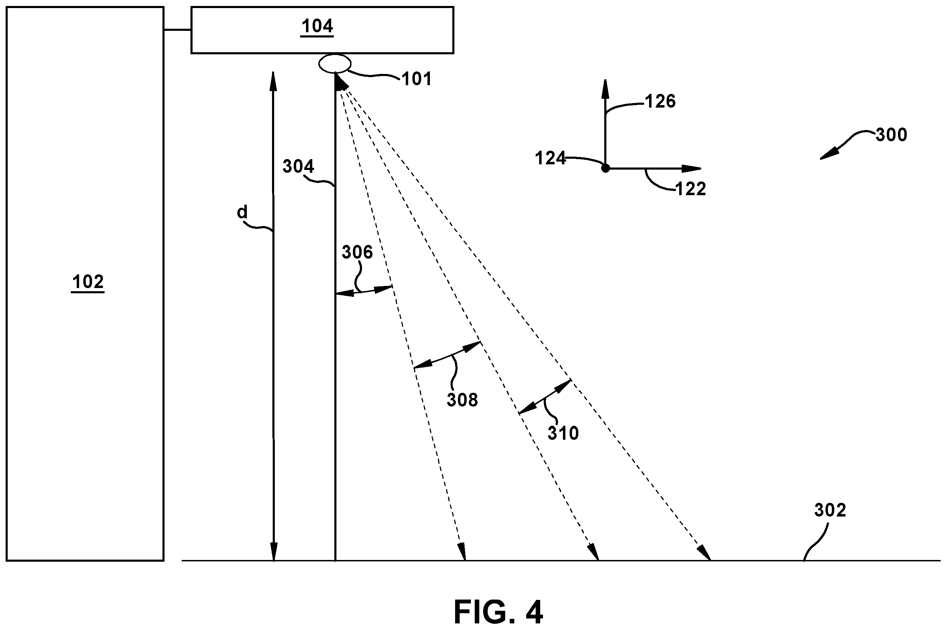

Referring now to FIGURE 4, there is a schematic diagram for arranging the one or more soil-density sensing devices 101 for use in the soil density sensing system 300. In this implementation, the soil-density sensing device 101 comprises a non-contact sensor mounted on the tow bar 104, as shown in FIGURE 1, a distance d above the ground 302 with respect to the vertical axis 126 such that the sensor does not contact the soil to detect soil density. Because non-contact sensors contain do not contact the soil (e.g., no friction on the moving parts), the sensors can provide consistent performance, are more resistant to dust and dirt than contact sensors, and the non-contact sensor can detect a wider area (e.g., greater coverage) than a contact sensor to produce a more encompassing profile of soil density throughout a target area. The non-contact sensor is also disposed in front of the row unit 102 with respect to the direction of travel substantially parallel to the longitudinal axis 122 for reasons that are described in greater detail below.

In this implementation, the non-contact sensor is configured to detect soil density throughout an area of interest (e.g., target area) defined by a range of detection angles, or viewing angles, measured relative to a line normal 304 to the ground 302 and tow bar 104. That is, for example, the angle of detection (e.g., the angle at which the detection signal is emitted by the sensor) can be determinative of the distance in front of the sensor encompassing the target area. In this way, for example, based on the ground speed of the vehicle, the downforce applicator may be provided time enough to act upon the detected soil density (e.g., apply more less or same force).

As an example, soil density data collected over an angular range of 0°-15° relative to the normal line 304, represented by 306, may be too close to the soil-density sensing device 101 and/or row unit 102 to provide sufficient time (e.g., notice) to make adjustments in real time based on the soil density data, such as by adjusting an actuator of a row unit to change the downward force exerted by the row unit on the soil. As an example, in some implementations, soil density data collected over an angular range of 15°-25° relative to the normal line 304, represented by 308, can provide accurate and reliable data combined with sufficient time (e.g., based on ground speed of the vehicle) to be proactive, such as adjusting row unit downforce (e.g. reducing downforce) based on the data, to mitigate a high downforce strike with a stone detected in the soil and/or to account for the more force required to penetrate higher density soil. As a further example, soil density data collected over an angular range greater than 25° (e.g., 25°-60°) relative to the normal line 304, represented by 310, may be too distant from the soil-density sensing device 101 and/or row unit 102 to provide accurate and reliable data to be proactive with the data, such as by adjusting the downward force of the row unit as will be explained in more detail below.

The soil density detection system 300 can be implemented with any type of non-contact sensor capable of penetrating the soil to detect variations in soil density throughout an area of interest (e.g., a target area). As an example, the non-contact sensor that generates the signal or image can comprise a ground-penetrating device that is configured to detect variations in soil density of a soil type (e.g., silt soil) as well as variations in soil density between different types of soil such as, for example, sandy soil and clay soil. In some implementations, the one or more non-contact sensors can be configured to detect the presence of objects in the soil that can affect soil density. As an example, the non-contact sensors can be configured to detect the presence of an air pocket in the soil and/or stone in the soil.

In some implementations, the one or more non-contact sensors can comprise one or more of a radio frequency (RF) sensor, ultrasound sensor, light detection and ranging (LIDAR) sensor, magnetic resonance sensor, ground penetrating radar (GPR), X-ray, or similar sensors that use ground penetrating signal to detect soil conditions. As an example, the non-contact sensor can comprise an RF sensor set to an operating frequency that can penetrate the soil to detect variations in soil density over an area in which the row unit is targeted to operate.

As an illustrative example, it can be beneficial for soil density data to be collected throughout an area covered by a desired angular range that the row unit has yet to traverse. But, the soil density sensing system 300 of this disclosure does not limit the positioning of the one or more non-contact sensors to a particular location, machine, and/or implement. Instead, the one or more soil-density sensing devices 101, comprising non-contact sensors, can be mounted, by way of non-limiting example, on one or more of the row unit 102, the rear tow bar 106, forward tow bar 104 (e.g., a 7x7 toolbar link for pulling an implement), work vehicle 108, and/or towing vehicle (e.g., tractor).

The system can comprise one or more soil-density sensing devices 101 (e.g., non-contact sensors) arranged to evaluate soil density prescribed detection angle. The one or more noncontacting sensors can be positioned to essentially 'look ahead' of the planter row unit, such as by being provided on the front portion of the row unit, as the row unit traverses a field for reasons that will be described in more detail below.

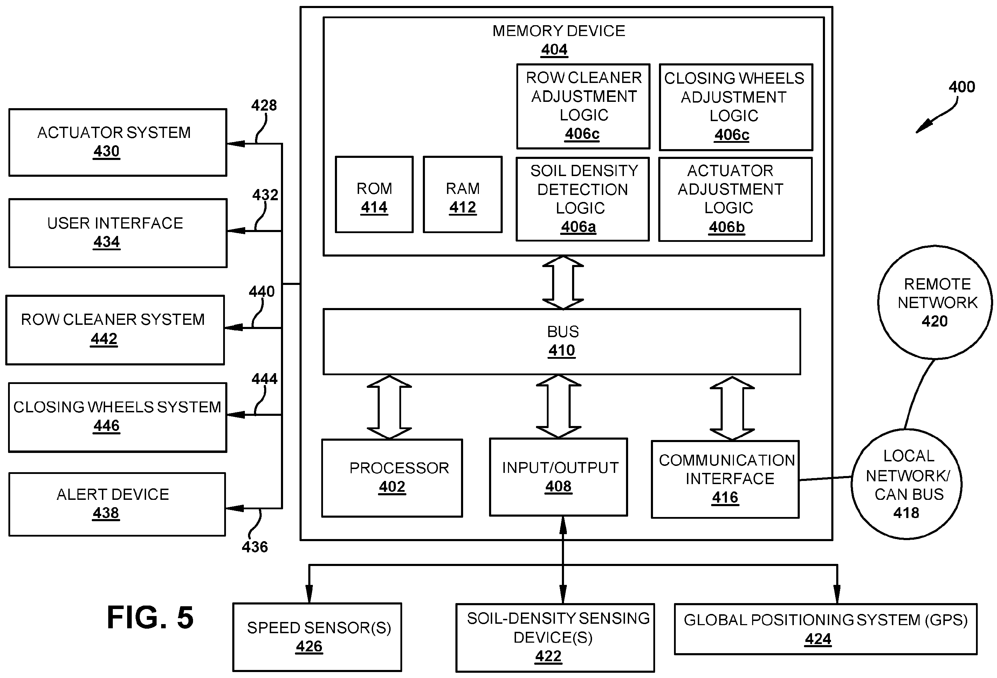

FIGURE 5 is a block diagram of a control device 400 or control unit, microprocessor, or the like, used in the soil-density detection system (e.g., 300 in FIGURE 4) and configured to determine variations in soil density throughout an area of interest based on sensed soil density data. The control device 400, such as a programmable controller, is suitable for executing implementations of one or more software systems, programs, or modules (programming) that are executable to provide a soil density detection system (e.g., 100, 300 in FIGURES 1 and 4 respectively) and method for determining myriad soil density conditions in the target area.

The control device 400 comprises a processor 402 (e.g., CPU, data processor), a memory device 404 (e.g., data memory storage, such as ROM, RAM memory, flash, solid state, etc.), that is operatively coupled with the processor 402, and executable instructions (e.g., programming or logic) stored in the memory device 404 such as, for example, soil density detection logic 406a, actuator adjustment logic 406b, closing wheel adjustment logic 406c, and row cleaner adjustment logic 406d collectively at 406. The control device 400 comprises an input/output 408 for receiving input from the one or more feedback devices, or sensors, and other systems as described in more detail below.

The control device 400 can include or be coupled with a bus 410 or other communication mechanism for communicating information and a processor 402 coupled with the bus 410 for processing information. In some implementation, the control device 400 can include a main memory 404, which may comprise random access memory (RAM) 412 or other dynamic storage devices for storing information and instructions such as soil density detection logic 406a and actuator adjustment logic 406b to be executed by the processor 402, and read only memory (ROM) 414 or other static storage device for storing static information and instructions for the processor 402. For example, the main memory 404 may be a non-volatile memory device and operable to store information and instructions executable by the processor 402.

The control device 400 can be operably coupled with one or more soil-density sensing devices 422 that are configured to detect soil density throughout an area of interest (e.g., target area). The one or more soil-density sensing devices 422 can be strategically disposed on the agricultural implement to detect soil density and to generate data indicative of soil density that can be translated to a signal and/or image (e.g., soil density profile or map) representative of the detected soil density. The soil-density sensing devices 422 can comprise non-contact sensors. In some implementations, one or more soil-density sensing devices 422 can be disposed in one or more sensor banks disposed on the implement or vehicle.

In some implementations, the control device 400 can be operably coupled with a global positioning system (GPS), or the like, 424 that is configured to provide a GPS signal indicative of location. In some implementations, the control device 400 can be operably coupled with one or more speed sensors 426 configured to detect the ground speed of the of the row unit as it traverses a field. The control device 400 may also be operably coupled with an actuator system 430 comprising one or more actuators.

In some implementations, the control device 400 can be operably coupled with an actuator system 430 comprising one or more actuators (e.g., 212 in FIGURE 2). The one or more actuators can be configured to adjust the downward force of the row unit against the soil. In these implementations, the control device 400 can provide an actuator adjustment command 428 based at least upon the data indicative of the soil density detected throughout the area of interest (e.g., target area). The downward force of the row unit can be adjusted by adjusting the one or more actuators in situ in response to the one or more actuator adjustment commands. In the alternative, the control device 400 can be operably coupled with one or more actuators (e.g., 212 in FIGURE 2) to control the downward force exerted by the row unit based at least upon the data indicative of the soil density detected throughout the area of interest.

In this implementation, the control device 400, such as by using the soil density detection logic 406a and actuator adjustment logic 406b collectively at 406, can identify an amount of downward force to apply to the row unit in order reach a desired soil depth based on the soil density data detected by the soil-density sensing devices 422. That is, for example, the amount of downforce to apply to the implement to reach a certain depth in soil of a known density may be based on a pre-determined relationship that is stored in the memory device 404 of the control device 400. The control device 400 can use the logic 406a, 406b to set the amount of downforce to apply to the row unit using one or more actuators to maintain a soil depth for the ground working tools based on the soil density data provided by the soil-density sensing device 422. When the soil density changes, such as when moving from low density soil (e.g., sand) to high density soil (e.g., that has been compacted from machinery), the soil-density sensing device data can provide an indication to the control device 400 to adjust the downward force applied to the row unit by sending an actuator adjustment command to the actuator system. In this implementation, the ground speed sensor 426 can provide data to the control device 400 that is indicative of the speed of the vehicle, which allows the processor to determine when the appropriate amount of force should be applied to account for the detected soil density.

In some implementations, the control device 400 can be operably coupled with a row cleaner system 442 comprising one or more row cleaners, or clearing wheels (e.g., 216 in FIGURE 3). The one or more row cleaners can be adjusted depending on the amount of fluff on top of the soil, where the row cleaners are operated at an increase intensity in higher density soil and a lower intensity in lower density soil. In these implementations, the control device 400 can use the row cleaner adjustment logic 406d to determine an amount of intensity for the at least one clearing wheel to work the soil. The control device 400 can provide a row cleaner adjustment command 440 based at least upon the data indicative of the soil density detected throughout the area of interest (e.g., target area). The intensity of the row cleaners can be adjusted (e.g., in real-time) in response to the row cleaner adjustment command 440.

In some implementations, the control device 400 can be operably coupled with a closing wheel system 446 comprising one or more sets of closing wheels (e.g., 220 in FIGURE 3) configured to close a furrow. The closing pressure of the one or more closing wheels can be adjusted to account for variations in soil density and soil type (e.g., clay soil versus sandy soil), which can be identified by the detected soil density. In these implementations, the control device 400 can use the closing wheels adjustment logic 406c to determine an amount of closing pressure for the closing wheels to apply to the soil. The control device 400 can provide a closing wheels adjustment command 444 based at least upon the data indicative of the soil density detected throughout the area of interest (e.g., target area). The closing pressure of the closing wheels can then be adjusted in real-time in response to receiving the closing wheels adjustment command 444.

In some implementations, the control device 400 can be operably coupled with a user interface 434, for example, comprising a user input and a user display (e.g., interactive display). In these implementations, the control device 400 can provide a soil density profile or map 432 based at least upon the data indicative of the soil density detected throughout the area of interest (e.g., presented in terms of geographical coordinates from the GPS system 424). The user interface 434 can display information to a user that helps the user identify status of the implement such as, for example, depth of the ground working tools (e.g., 210 in FIGURE 3), soil condition information, soil density information, identifying objects in the soil (e.g., a rock or air pocket), the amount of actuator force applied to the row unit, etc. In some implementations, the user interface can be visible to an operator within the cab of a tractor. Further, the user interface 434 may allow the user to input instruction (e.g., via a user input) regarding adjustment of the amount of downward force (e.g., or upward force) applied to the implement (e.g., a row unit).

In some implementations, the control device 400 can be communicatively coupled with an alert device 438. In these implementations, the control device 400 may also generate an alert signal 436 to activate the alert device 438 such as, for example, an audible alarm and/or flashing light to alert the operator of an approaching hazard. As an example, the control device 400 may generate an alert signal 436 if a stone has been detected in the travel path of a size and composition that, if contacted by the ground working tools of the row unit, may damage the tools. The alert device 438 then alerts the operator to the approaching stone hazard so that the operator and/or machine (e.g., automated corrective response) can reduce the row unit downforce by decreasing the actuator force and/or maneuvering around the detected stone.

The control device 400 comprises a communication interface 416 that can be configured to report collected soil density data, and any soil density profiles and/or maps generated from said collected soil density data, to a local network/CAN bus 418. A remote network 420 can be communicatively coupled with the local network/CAN bus 418 to share soil density and for remote storage of the soil density profiles and maps. Soil density data collected over time can be used for mapping purposes to document changing soil density conditions over time for reasons that include environmental factors (e.g., wind and water transport soil over time) and human influence (e.g., human use). As an example, a soil map could be prepared for clay soil, sandy soil and silt soil over time, with the results of compared and tied together for use as an input to the tillage pass.

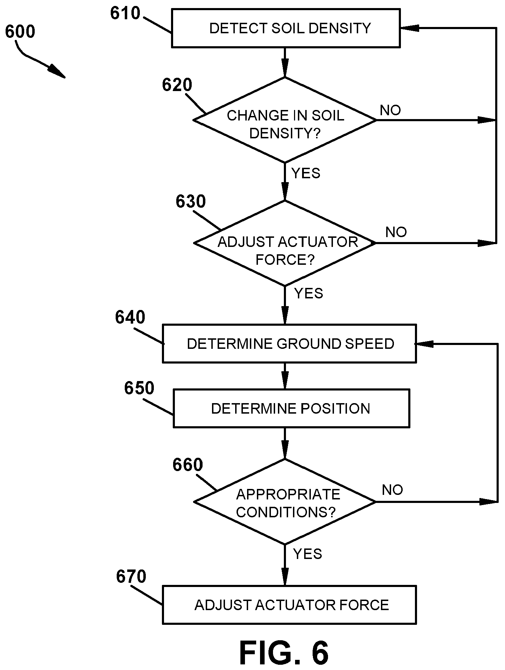

Referring now to FIGURE 6, there is a flow diagram illustrating an example implementation of an exemplary method 600 for adjusting downward force of the row unit based upon detected soil density. The method 600 is implemented using the soil density detection system of this disclosure, which can be configured as a closed loop feedback system having wherein soil density data is continuously detected and analyzed to make adjustments during operation (e.g., in real time) based at least on the detected soil density data.

At 610, the method comprises detecting soil density in an area of interest using one or more soil-density sensing devices. The soil-density sensing devices can comprise non-contact sensors that are configured to detect soil density in an area of interest (e.g., target area) and generate data that can be used to generate a signal or image (e.g., soil density profile) representative of the detected soil density.

At 620, it is determined whether the detected soil density data received from one or more soil-density sensing devices indicates a change in soil density. For example, soil density can change as the row unit traverses a field based on the type of soil encountered by the row unit and may be the result of soil compaction, or differing soil conditions. If there is no change in soil density, the control device may make no adjustment to the actuator force and the actuator force remains at its current setting. If there is a change in soil density, the control device can determine (e.g., based on received data and a stored program) how the actuator force can be adjusted to account for the change in density. For example, if an increase in soil density is detected, the soil is may more difficult to penetrate with ground working tools and, as a result, more force may be needed to achieve a same, targeted soil depth. Conversely, if soil density decreases, the soil may be less difficult to penetrate with ground working tools and, as a result, less force may be needed to reach the target depth.

For example, detecting an increase in soil density can result in identifying an amount to increase actuator force based at least upon the soil density data indicative of the soil density detected in the area of interest (e.g., target area). Further, detecting a decrease in soil density may result in identifying an amount to decrease actuator force based at least upon the data indicative of the soil density detected throughout the area of interest (e.g., target area).

Once the amount of increase or decrease (e.g., or no change) is determined, other factors may be considered when determining the appropriate downward force to be applied by the row unit to account for the soil condition (e.g., soil having the density that matches the adjustment to the actuator force) at the appropriate time (e.g., when the row unit is accessing the soil). At 640, the ground speed of the row unit traversing a field is determined. At 650, the position of the row unit in relation to the soil having the respective density is determined.

At 660, it is determined whether the conditions are met to adjust the actuator force. For example, if the row unit is disposed over the area of soil having a density that matches the intended adjustment to the actuator force, then the condition is met.

At 670, the actuator force can be adjusted. For example, an actuator adjustment command can be provided to an actuator system comprising one or more actuators configured to adjust the downward force of the row unit against the soil. The actuator adjustment command can be based at least upon the data indicative of the soil density detected throughout the area of interest (e.g., target area), along with ground speed, position detection, and other factors. The system and method of the present disclosure offers several advantages. First, by using one or more soil-density sensing devices comprising non-contact sensors, the sensors may not be affected by wear, which can mitigate mechanical failure. Moreover, the non-contact sensors provide advantages because they have a longer service life, fast response, are highly reliable and dependable, and because they do use contact for detection of soil conditions, can be arranged to look ahead of the row unit and detect soil density over a wider area and before the row unit encounters the soil. Further, by providing soil density data before the row unit encounters the soil, the operator and/or the machine can make adjustments during operation (e.g., in real-time) such as by adjusting the actuator force to maintain planting depth or to avoid a hard strike on stone, or maneuvering the implement to avoid stone.

The implementations have been described, hereinabove. It will be apparent to those skilled in the art that the above methods and apparatuses may incorporate changes and modifications without departing from the scope of this invention. It is intended to include all such modifications and alterations in so far as they come within the scope of the appended claims.

## Claims

1. Combination of a soil density detection system (100, 300) and an agricultural implement, comprising:
at least one non-contact soil-density sensing device (101, 422) disposed on the agricultural implement, the soil-density sensing device (101, 422) operably detecting soil density in at least a portion of a target area in front of a ground contact portion of said agricultural implement to generate data indicative of a detected soil density; wherein the soil-density sensing device (101, 422) is configured to detect soil density in the target area defined by a range of detection angles measured relative to a line normal (304) to the ground (302), wherein the detection angle range is 15°-25°, inclusive, relative to the normal line (304);
a control device (400) operatively coupled with the at least one soil-density sensing device (101), the control device (400) comprising:
a processor (402) for processing data and programming;
a memory device (404) operatively coupled with the processor (402) to operably store data and programming; and
soil density detection logic (406a) stored as programming in the memory device (404), the soil density detection logic (406a) being executable by the processor (402) to determine the soil density throughout the target area based at least upon the sensed soil density data,
wherein the control device (400) is configured to generate data indicative of the soil density detected throughout the target area,
wherein the control device (400) is operably coupled with a user interface (434) configured to display a soil density profile or map based at least upon the data indicative of the soil density detected in the target area, and
wherein the control device (400) is operably coupled with a global positioning system (GPS) (424) for determining position data.

2. The combination of claim 1, wherein the control device (400) is operably coupled with an actuator system (430) comprising one or more actuators (212) configured to adjust actuation force on the ground contact portion of the agricultural implement.

3. The combination of claim 2, wherein the control device (400) further comprises actuator adjustment logic (406b) stored as programming in the memory device (404), the actuator adjustment logic (406b) being executable by the processor (402) to determine an amount of actuator force to apply to ground contact portion of the agricultural implement based at least upon the data indicative of the soil density detected throughout the target area.

4. The combination of claim 3, wherein the control device (400) is configured to provide an actuator adjustment command (428) to the actuator system (430) based at least upon the determined amount of actuator force to apply to the agricultural implement, the actuator adjustment command (428) indicative of the amount of actuator force to apply to the agricultural implement.

5. The combination of one of the claims 1 to 4, wherein the control device (400) is operably coupled with a closing wheels system (446) comprising at least one set of closing wheels (220) configured to close a furrow.

6. The combination of claim 5, wherein the control device (400) further comprises closing wheel adjustment logic (406c) stored as programming in the memory device (404), the closing wheel adjustment logic (406c) being executable by the processor (402) to determine an amount of closing pressure for the closing wheels (220) to apply to the soil.

7. The combination of claim 6, wherein the control device (400) is configured to provide a closing wheels adjustment command (444) to the closing wheel system (446) based at least upon the data indicative of the soil density detected throughout the target area, the closing wheels adjustment command (444) indicative of the amount of closing pressure for the closing wheels (220) to apply to the soil.

8. The combination of one of the claims 1 to 7, wherein the control device (400) is operably coupled with a row cleaner system (442) comprising at least one clear wheel (216) to work the soil.

9. The combination of claim 8, wherein the control device (400) further comprises row cleaner adjustment logic (406d) stored as programming in the memory device (404), the row cleaner adjustment logic (406d) being executable by the processor (402) to determine an amount of speed and/or force used for the at least one clearing wheel (216) to work the soil.

10. The combination of claim 9, wherein the control device (400) is configured to provide a row cleaner adjustment command (440) to the row cleaner system (442) based at least upon the data indicative of the soil density detected throughout the target area, the row cleaner adjustment command (440) indicative of the amount of speed or force used for the clearing wheel (216) to work the soil.

11. The combination of one of the claims 1 to 10, wherein the non-contact soil-density sensing device (101, 422) comprises at least one non-contact sensor comprising one or more of: a radio frequency (RF) sensor, ultrasound sensor, light detection and ranging (LIDAR) sensor, magnetic resonance sensor, ground penetrating radar (GPR), and X-ray detector.

## Patentansprüche

1. Kombination aus einem Bodendichtedetektionssystem (100, 300) und einem landwirtschaftlichen Arbeitsgerät, umfassend:
mindestens eine berührungslose Bodendichteerfassungsvorrichtung (101, 422), die auf dem landwirtschaftlichen Arbeitsgerät angeordnet ist, wobei die Bodendichteerfassungsvorrichtung (101, 422) die Bodendichte in mindestens einem Abschnitt eines Zielbereichs vor einem Bodenkontaktabschnitt des landwirtschaftlichen Arbeitsgeräts operativ detektiert, um Daten zu erzeugen, die eine detektierte Bodendichte angeben; wobei die Bodendichteerfassungsvorrichtung (101, 422) dazu ausgelegt ist, eine Bodendichte in dem Zielbereich zu detektieren, der durch einen Bereich von Detektionswinkeln definiert ist, die relativ zu einer Linie normal (304) zum Boden (302) gemessen werden, wobei der Detektionswinkelbereich 15°-25°, einschließlich, relativ zu der Normallinie (304) beträgt;
eine Steuervorrichtung (400), die operativ mit der mindestens einen Bodendichteerfassungsvorrichtung (101) gekoppelt ist, wobei die Steuervorrichtung (400) Folgendes umfasst:
einen Prozessor (402) zur Verarbeitung von Daten und Programmierung;
eine Speichervorrichtung (404), die operativ mit dem Prozessor (402) gekoppelt ist, um Daten und eine Programmierung operativ zu speichern; und
eine Bodendichtedetektionslogik (406a), die als Programmierung in der Speichervorrichtung (404) gespeichert ist, wobei die Bodendichtedetektionslogik (406a) durch den Prozessor (402) ausführbar ist, um die Bodendichte im gesamten Zielbereich basierend zumindest auf den erfassten Bodendichtedaten zu bestimmen,
wobei die Steuervorrichtung (400) dazu ausgelegt ist, Daten zu erzeugen, die die Bodendichte angeben, die im gesamten Zielbereich detektiert wird,
wobei die Steuervorrichtung (400) operativ mit einer Benutzerschnittstelle (434) gekoppelt ist, die dazu ausgelegt ist, ein Bodendichteprofil oder eine Bodendichtekarte basierend zumindest auf den Daten, die die in dem Zielbereich detektierte Bodendichte angeben, anzuzeigen, und
wobei die Steuervorrichtung (400) mit einem globalen Positionierungssystem (GPS) (424) zum Bestimmen von Positionsdaten operativ gekoppelt ist.

2. Kombination nach Anspruch 1, wobei die Steuervorrichtung (400) operativ mit einem Aktuatorsystem (430) gekoppelt ist, das einen oder mehrere Aktuatoren (212) umfasst, die dazu ausgelegt sind, eine Betätigungskraft auf den Bodenkontaktabschnitt des landwirtschaftlichen Arbeitsgeräts einzustellen.

3. Kombination nach Anspruch 2, wobei die Steuervorrichtung (400) ferner eine Aktuatoreinstelllogik (406b) umfasst, die als Programmierung in der Speichervorrichtung (404) gespeichert ist, wobei die Aktuatoreinstelllogik (406b) durch den Prozessor (402) ausführbar ist, um einen Betrag einer Aktuatorkraft, die auf den Bodenkontaktabschnitt des landwirtschaftlichen Arbeitsgeräts anzuwenden ist, basierend zumindest auf den Daten, die die Bodendichte angeben, die im gesamten Zielbereich detektiert wird, zu bestimmen.

4. Kombination nach Anspruch 3, wobei die Steuervorrichtung (400) dazu ausgelegt ist, dem Aktuatorsystem (430) zumindest basierend auf dem bestimmten Betrag an Aktuatorkraft, der auf das landwirtschaftliche Arbeitsgerät anzuwenden ist, einen Aktuatoreinstellbefehl (428) bereitzustellen, wobei der Aktuatoreinstellbefehl (428) den Betrag an Aktuatorkraft angibt, der auf das landwirtschaftliche Arbeitsgerät anzuwenden ist.

5. Kombination nach einem der Ansprüche 1 bis 4, wobei die Steuervorrichtung (400) operativ mit einem Schließradsystem (446) gekoppelt ist, das mindestens einen Satz von Schließrädern (220) umfasst, die dazu ausgelegt sind, eine Furche zu schließen.

6. Kombination nach Anspruch 5, wobei die Steuervorrichtung (400) ferner eine Schließradeinstellungslogik (406c) umfasst, die als Programmierung in der Speichervorrichtung (404) gespeichert ist, wobei die Schließradeinstellungslogik (406c) durch den Prozessor (402) ausführbar ist, um einen Betrag eines Schließdrucks für die Schließräder (220) zu bestimmen, der auf den Boden anzuwenden ist.

7. Kombination nach Anspruch 6, wobei die Steuervorrichtung (400) dazu ausgelegt ist, dem Schließradsystem (446) zumindest basierend auf den Daten, die die Bodendichte angeben, die im gesamten Zielbereich detektiert wird, einen Schließradeinstellbefehl (444) bereitzustellen, wobei der Schließradeinstellbefehl (444) den Betrag an Schließdruck angibt, den die Schließräder (220) auf den Boden anwenden sollen.

8. Kombination nach einem der Ansprüche 1 bis 7, wobei die Steuervorrichtung (400) operativ mit einem Reihenreinigersystem (442) gekoppelt ist, das mindestens ein Räumrad (216) zum Bearbeiten des Bodens umfasst.

9. Kombination nach Anspruch 8, wobei die Steuervorrichtung (400) ferner eine Reihenreinigereinstelllogik (406d) umfasst, die als Programmierung in der Speichervorrichtung (404) gespeichert ist, wobei die Reihenreinigereinstelllogik (406d) durch den Prozessor (402) ausführbar ist, um einen Betrag an Drehzahl und/oder Kraft zu bestimmen, den das mindestens eine Räumrad (216) zum Bearbeiten des Bodens verwendet.

10. Kombination nach Anspruch 9, wobei die Steuervorrichtung (400) dazu ausgelegt ist, dem Reihenreinigersystem (442) zumindest basierend auf den Daten, die die Bodendichte angeben, die im gesamten Zielbereich detektiert wird, einen Reihenreinigereinstellbefehl (440) bereitzustellen, wobei der Reihenreinigereinstellbefehl (440) den Betrag an Geschwindigkeit oder Kraft angibt, den das Räumrad (216) zum Bearbeiten des Bodens verwendet.

11. Kombination nach einem der Ansprüche 1 bis 10, wobei die berührungslose Bodendichteerfassungsvorrichtung (101, 422) mindestens einen berührungslosen Sensor umfasst, der eines oder mehrere von Folgendem umfasst: einen Hochfrequenz(HF)-Sensor, einen Ultraschallsensor, einen Lichtdetektions- und Entfernungsmessungssensor (LIDAR), einen Magnetresonanzsensor, ein Bodenradar (GPR) und einen Röntgendetektor.

## Revendications

1. Combinaison d'un système de détection de densité de terre (100, 300) et d'un outil agricole, comprenant :
au moins un dispositif de détection de densité de terre sans contact (101, 422) disposé sur l'outil agricole, le dispositif de détection de densité de terre (101, 422) détectant de façon exploitable une densité de terre dans au moins une portion d'une zone cible devant une portion de contact avec le sol dudit outil agricole pour générer des données indicatives d'une densité de terre détectée ;
dans laquelle le dispositif de détection de densité de terre (101, 422) est configuré pour détecter une densité de terre dans la zone cible définie par une plage d'angles de détection mesurés relativement à un trait (304) normal au sol (302), dans laquelle la plage d'angle de détection est de 15° à 25°, inclus, relativement au trait normal (304) ;
un dispositif de commande (400) couplé opérationnellement à l'au moins un dispositif de détection de densité de terre (101), le dispositif de commande (400) comprenant :
un processeur (402) pour traiter des données et une programmation ;
un dispositif de mémoire (404) couplé opérationnellement au processeur (402) pour stocker de façon exploitable des données et une programmation ; et
une logique de détection de densité de terre (406a) stockée sous forme de programmation dans le dispositif de mémoire (404), la logique de détection de densité de terre (406a) étant exécutable par le processeur (402) pour déterminer la densité de terre dans la totalité de la zone cible sur la base au moins des données de densité de terre détectée,
dans laquelle le dispositif de commande (400) est configuré pour générer des données indicatives de la densité de terre détectée dans la totalité de la zone cible,
dans laquelle le dispositif de commande (400) est couplé opérationnellement à une interface utilisateur (434) configurée pour afficher un profil ou une carte de densité de terre sur la base au moins des données indicatives de la densité de terre détectée dans la zone cible, et
dans laquelle le dispositif de commande (400) est couplé opérationnellement à un système mondial de positionnement (« Global Positioning System », GPS) (424) pour déterminer des données de position.

2. Combinaison de la revendication 1, dans laquelle le dispositif de commande (400) est couplé opérationnellement à un système d'actionneur (430) comprenant un ou plusieurs actionneurs (212) configurés pour régler une force d'actionnement sur la portion de contact avec le sol de l'outil agricole.

3. Combinaison de la revendication 2, dans laquelle le dispositif de commande (400) comprend en outre une logique de réglage d'actionneur (406b) stockée sous forme de programmation dans le dispositif de mémoire (404), la logique de réglage d'actionneur (406b) étant exécutable par le processeur (402) pour déterminer une quantité de force d'actionneur à appliquer sur la portion de contact avec le sol de l'outil agricole sur la base au moins des données indicatives de la densité de terre détectée dans la totalité de la zone cible.

4. Combinaison de la revendication 3, dans laquelle le dispositif de commande (400) est configuré pour fournir une instruction de réglage d'actionneur (428) au système d'actionneur (430) sur la base au moins de la quantité déterminée de force d'actionneur à appliquer sur l'outil agricole, l'instruction de réglage d'actionneur (428) étant indicative de la quantité de force d'actionneur à appliquer sur l'outil agricole.

5. Combinaison d'une des revendications 1 à 4, dans laquelle le dispositif de commande (400) est couplé opérationnellement à un système de roues de fermeture (446) comprenant au moins un ensemble de roues de fermeture (220) configurées pour fermer un sillon.

6. Combinaison de la revendication 5, dans laquelle le dispositif de commande (400) comprend en outre une logique de réglage de roues de fermeture (406c) stockée sous forme de programmation dans le dispositif de mémoire (404), la logique de réglage de roues de fermeture (406c) étant exécutable par le processeur (402) pour déterminer une quantité de pression de fermeture pour les roues de fermeture (220) à appliquer sur la terre.

7. Combinaison de la revendication 6, dans laquelle le dispositif de commande (400) est configuré pour fournir une instruction de réglage de roues de fermeture (444) au système de roues de fermeture (446) sur la base au moins des données indicatives de la densité de terre détecté dans la totalité de la zone cible, l'instruction de réglage de roues de fermeture (444) étant indicative de la quantité de pression de fermeture pour les roues de fermeture (220) à appliquer sur la terre.

8. Combinaison d'une des revendications 1 à 7, dans laquelle le dispositif de commande (400) est couplé opérationnellement à un système de nettoyeuse de ligne (442) comprenant au moins une roue de défrichement (216) pour travailler la terre.

9. Combinaison de la revendication 8, dans laquelle le dispositif de commande (400) comprend en outre une logique de réglage de nettoyeuse de ligne (406d) stockée sous forme de programmation dans le dispositif de mémoire (404), la logique de réglage de nettoyeuse de ligne (406d) étant exécutable par le processeur (402) pour déterminer une quantité de vitesse et/ou force utilisée pour l'au moins un roue de défrichement (216) pour travailler la terre.

10. Combinaison de la revendication 9, dans laquelle le dispositif de commande (400) est configuré pour fournir une instruction de réglage de nettoyeuse de ligne (440) au système de nettoyeuse de ligne (442) sur la base au moins des données indicatives de la densité de terre détectée dans la totalité de la zone cible, l'instruction de réglage de nettoyeuse de ligne (440) étant indicative de la quantité de vitesse ou de force utilisée pour la roue de défrichement (216) pour travailler la terre.

11. Combinaison d'une des revendications 1 à 10, dans laquelle le dispositif de détection de densité de terre sans contact (101, 422) comprend au moins un capteur sans contact comprenant un ou plusieurs éléments parmi : un capteur radiofréquence (RF), un capteur ultrason, un capteur de détection et de télémétrie par lumière (« Light Detection and Ranging », LIDAR), un capteur à résonance magnétique, un radar de pénétration de sol (« Ground Penetrating Radar », GPR), et un détecteur à rayons X.
